Europäisches Patentamt

**European Patent Office**

Office européen des brevets

(11) Publication number: **0 122 614 B1**

# EUROPEAN PATENT SPECIFICATION

(45) Date of publication of patent specification: **14.07.93** (51) Int. Cl.5: **C12Q 1/68**

(21) Application number: **84104165.0**

(22) Date of filing: **12.04.84**

(54) **Kit for terminally chemically labeling DNA.**

(30) Priority: **13.04.83 US 484583**

(43) Date of publication of application:
**24.10.84 Bulletin 84/43**

(45) Publication of the grant of the patent:
**14.07.93 Bulletin 93/28**

(84) Designated Contracting States:
**AT BE CH DE FR GB IT LI LU NL SE**

(56) References cited:
**EP-A- 0 063 879**
**EP-A- 0 097 373**
**EP-A- 0 117 440**
**WO-A-83/02277**
**US-A- 4 307 189**

**CHEMICAL ABSTRACTS, vol. 93, no. 13, 29th
September 1980, page 211, no. 127333m, Co-
lumbus, Ohio, US; S.N. COHEN et al.: "3'-End
labeling of DNA with
[alpha-32P]-cordycepin-5'-triphosphate", &
GENE 1980, 10(2), 177-83**

**Maniatis et al. (1982) "Molecular Cloning"**

(73) Proprietor: **ENZO BIOCHEM, INC.**
**325 Hudson Street**
**New York, N.Y. 10013(US)**

(72) Inventor: **Brakel, Christine L.**
**530 Ackerson Boulevard**
**Brightwaters New York(US)**
Inventor: **Stavrianopoulos, Jannis G.**
**515 West 59 Street**
**New York New York(US)**

(74) Representative: **Vossius & Partner**
**Siebertstrasse 4 P.O. Box 86 07 67**
**W-8000 München 86 (DE)**

**Description**

Terminal deoxynucleotidyl transferase is known and has been employed to catalyze the polymerization of deoxynucleoside triphosphates for the elongation of polydeoxynucleotide chains or molecules (DNA). The utilization and effectiveness of terminal deoxynucleotidyl transferase is described by F. J. Bollum in the article entitled "Terminal Deoxynucleotidyl Transferase", in the publication The Enzymes, (P.D. Boyer, ed.), 3rd Ed. Vol. 10, pp. 145-171, Academic Press, New York, N.Y. (1974). The disclosures of this publication are herein incorporated and made part of this disclosure.

Another publication of interest with respect to terminal transferase (terminal deoxynucleotidyl transferase) and its utilization for the addition of homopolymers to the 3' ends of DNA is the article by T. Nelson and D. Brutlag entitled "Addition of Hompolymers to the 3'-Ends of Duplex DNA with Terminal Transferase", which appeared in Methods in Enzymology, Vol. 68, pp. 41-50, Academic Press (1979). Also of interest with respect to the utilization of terminal nucleotidyl transferase is the article by C. Vincent, P. Tchen, M. Cohen-Solal and P. Kourilsky entitled "Synthesis of 8-(2-4 dinitrophenyl 2-6 aminohexyl) amino-adenosine 5' triphosphate: biological properties and potential uses", which appeared in Nucleic Acids Research, Vol. 10, No. 21, pp.6787-6796, published by IRL Press Limited, Oxford, England (1982). The above-identified article by Vincent et al discloses that the above-entitled compound is a substrate for calf thymus deoxynucleotidyl terminal transferase and that this compound can be incorporated into DNA molecules by elongation from 3' ends and that the incorporated nitrophenyl group can be recognized by specific antibodies which can then be detected by anti-antibodies coupled to an enzyme. If desired, the dinitrophenyl group can be introduced into DNA after enzymatic incorporation of 8-aminohexyl adenosine 5' triphosphate and reaction with 1-fluoro-2-4-nitrobenzene. The disclosures of this article are herein incorporated and made part of this disclosure.

Of additional interest is the article by P. R. Langer, A. A. Waldrop & D.C. Ward entitled "Enzymatic synthesis of biotin-labeled polynucleotides: Novel nucleic acid affinity probes", Proc. Natl. Acad. Sci., Vol. 78, No. 11, pp. 6633-6637, November 1981, which discloses certain biotinylated nucleotides capable of incorporation in double-stranded DNA and useful as substrates for a variety of DNA and RNA polymerases. It is suggested in this article that the disclosed biotin-labeled polynucleotides would be useful as affinity probes for the detection and isolation of specific DNA and RNA sequences. The disclosure of this publication is also incorporated in and made part of this disclosure.

Of special interest related to the practices of this invention is U.S. Patent 4,358,535 which issued to S. Falkow, and S. Mosley entitled "Specific DNA Probes in Diagnostic Microbiology". Accordingly, the disclosures of the Falkow et al U.S. Patent 4,358,535 are also incorporated and made part of this disclosure.

It is an object of this invention to provide special terminally labeled DNA molecules useful as DNA probes.

It is another object of this invention to provide materials, reagents and techniques for the preparation of terminally chemically labeled DNA probes.

How these and other objects of this invention are accomplished will become apparent in the light of the accompanying disclosure.

The terminal chemical labeling of DNA molecules, such as DNA probes, is effected by employing terminal deoxynucleotide transferase (TdT). In the terminal chemical labeling of DNA molecules, there is employed in combination with TdT, as a substrate therefor, a chemically labeled nucleotide, such as a biotinylated nucleotide, e.g. 2'-deoxyuridine triphosphate 5-allyl-amine-biotin also identified as biotin-11-dUTP, or other biotinylated nucleotides capable of acting as a substrate for TdT for terminal attachment to the DNA. In the practice of this invention any nucleotide, preferably chemically labeled, useful as a substrate for TdT and capable of terminal attachment via TdT to the DNA being labeled is usefully employed. Although it is desirable that the nucleotides so terminally attached to the DNA comprise at least one biotinylated nucleotide, it is desirable that the nucleotides terminally attached to the DNA consist of more than 50% by number biotinylated nucleotides. However, nucleotides terminally attached to the DNA via TdT may consist of or comprise only a minor portion or percentage, in the range from about 2% to about 40% by number, biotinylated nucleotides, i.e. biotinylated deoxyribonucleotides.

In accomplishment of the above, i.e. the terminal labeling of DNA probes, such as the end or terminal biotinylation of DNA probes via terminal deoxynucleotide transferase TdT, there are provided, in accordance with the practices of this invention, reagents and procedures employing the same for the in vitro preparation of DNA probes with high biotin content. By employing TdT, a polymer with a biotin-containing analog of TTP is formed on the 3'-OH terminus of the DNA of interest. In accordance with the practices of this invention, there are provided reagents, in kit form, for the synthesis of a non-radioactive, chemically-labeled stable DNA probe. The special nucleotides making up said probe are detectable by techniques, such as the use of anti-antibodies directed to an antibody attached to the terminally linked special nucleotides attached to the DNA probe or by the use of, for example, enzyme-linked avidin or streptavidin which would attach itself to the biotinylated special nucleotides terminally attached to the DNA probes.

In a kit provided for the practices of this invention, i.e. for the terminal labeling of DNA probes employing terminal deoxynucleotide transferase, the kit would contain the following components or reagents or tubes:

1. Terminal deoxynucleotide transferase
   20 unit/$\mu$l in 100 mM KCacodylate pH 7.0; 5mM 2-mercaptoethanol; 50% v/v glycerol
2. Terminal deoxynucleotide transferase dilution buffer
   50 mM KCacodylate pH 7.0;
   5mM 2-mercaptoethanol; 1mg/ml bovine serum albumin (enzyme stabilizer grade ERT-701).
3. DNase
   0.5mg/ml in 0.1M $MgCl_2$
4. DNase I dilution buffer
   10mM Tris HCl pH 7.5
   1mg/ml bovine serum albumin (ERT-701).
5. 3.3 x terminal labeling reaction buffer
   0.66M KCacodylate pH 7.0
   0.0033M 2-mercaptoethanol
6. Deoxynucleotide solutions
   a. 9mM dTTP
   b. 9mM dCTP
7. Bio Probe (Bio-dUTP) Solution
   2.5mM Bio dUTP
8. $CoCl_2$
   0.01M
9. 3H dTTP (40-60 Ci/mmol)
   [methyl-3H] Thymidine-5'-triphosphate ammonium salt in 50% aqueous ethanol, 0.25 $\mu$Ci./$\mu$l. (The 3H dTTP is used solely to monitor incorporation and is supplied by Amersham Corp.)
10. Control DNA, DNase treated
    0.5mg/ml in 50mM Tris HCl pH 7.4, 5mM $MgCl_2$, heat inactivated DNase. (to be used as terminal labeling control.)

A preferred procedure for the preparation of the DNA for terminal labeling employing the DNase component of a kit in accordance with this invention is as follows:

For general purposes, 3'OH termini introduced by DNase I are effective primer termini for terminal transferase. DNA is digested with DNase to a fragment size of 200-500 base pairs (or whatever size is desired). The DNase is subsequently inactivated by heat and the digested DNA is ready to be terminally labeled. Some variation in digestion will occur, and it is best to follow the general outline described here and then observe the size of the DNA on agarose (and/or acrylamide) gels.

In a plastic tube place
a. 5$\mu$g DNA in 5$\mu$l or less
b. 2.5$\mu$l 0.04M $MgCl_2$
c. $H_2O$ to give 8.0ul

To this tube add 2.0 $\mu$l of DNase freshly diluted in the following manner: 1$\mu$l DNase plus 99$\mu$l DNase dilution buffer; one microliter of this solution is then diluted with 49 $\mu$l DNase, dilution buffer. After addition of DNase the tube is incubated for 2 to 10 minutes (5 minutes is generally optimal) at 37ºC. and then for 5 minutes at 68ºC. to inactivate the DNase.

It is important to note that if the DNA solution is dilute and contains EDTA, and is concentrated by lyophilization prior to digestion, the concentration of EDTA must be accounted for so that the $MgCl_2$ concentration is at least 5mM during digestion.

After inactivating the DNase, the DNase treated DNA is ready for terminal labeling.

After treatment of the DNA by DNase to prepare the DNA for terminal labeling, the following procedure is employed:

A. Dilution of Terminal Transferase

Terminal transferase (tube 1) should be diluted prior to each use by adding 1.5 $\mu$l of TdT to 4.5 $\mu$l of TdT dilution buffer (tube 2).

3

B. Lyophilization of 3H dTTP

Lyophilize 8μl of 3H-dTTP in a plastic tube in which the reaction will be performed.

C. Terminal Labeling Reaction.

Upon removal of ethanol by lyophilization, the remaining reagents are added to 3H dTTP in the following order:

| Tube No. | Components | Volume Per Reaction |
|---|---|---|
| 5. | 3.3x TdT Reaction Buffer | 15μl |
| 6a or 6b | 9mM dTTP or 9mM dCTP | 5μl |
| | OR | |
| 6a + 6B | 9mM dTTP + 9mM dCTP | 2.5 μl 6a + 2.5μl 6b |
| 7. | 2.5mM Bio dUTP | 2μl |
| | Experimental DNA to be terminally labeled | 1μg |
| | OR | |
| | Control DNase-treated DNA | 2μl |

Adjust volume to 40μl with sterile distilled $H_2O$ before adding:

| | | |
|---|---|---|
| 1+2 | Diluted TdT | 5μl |
| 8. | $CoCl_2$ | 5μl |

Incubate the reaction at 37°C. for one hour. Chill reaction to 0°C. and determine incorporation (see below). If further incorporation is desired, continue to incubate the reaction at 37°C. Under most circumstances, sufficient incorporation should be obtained after 1-2 hours of incubation. Using the DNase introduced 3'OH primer termini, 10-30 nanomoles of total nucleotide are incorporated. Stop the reaction by heating at 65°C. for 5 minutes, or by adding 1/10 volume 0.1M EDTA.

D. Determination of Incorporated Nucleotide

1. Remove a 2 microliter aliquot into a 5 ml plastic tube to which 10 micrograms of poly rA has been added.
2. Add 1ml cold 5% (w/v) trichloroacetic acid (TCA), 25mM sodium pyrophosphate.
3. Keep tube in ice for at least 10 minutes.
4. Filter through glass fiber filters.
5. Wash filters thoroughly with 2.5% TCA, 10mM sodium pyrophosphate.
6. Dry thoroughly.
7. Add toluene-based liquid scintillation cocktail to cover the filter and count in a liquid scintillation counter.
8. To determine total radioactivity in the reaction mixture, transfer a second 2 microliter aliquot into 150 microliters directly (without filtration) on a glass fiber filter. Dry and count.

E. Calculation of nanomoles deoxynucleotide incorporated and estimation of biotinylated dUTP content:

Using the protocol described above and the reaction as described in part C, calculate the nanomoles of nucleotides incorporated by the following equation:

$$\text{Nanomoles nucleotide incorporated} = \frac{\text{cpm TCA precipitated}}{\mu g \text{ of DNA}} \times 50$$
$$\frac{}{} \quad \text{cpm total}$$

To estimate the content of the biotinylated dUTP per ug of probe DNA, multiply the total incorporated nucleotides by 0.10 (the fraction of Bio-dUTP in reaction).

The amount of Bio dUTP per $\mu$g of DNA should be from 1-3 nanomoles.

F. Recovery of Biotinylated DNA:

1. Load "stopped" terminal labeling reaction mixture on a Sephadex G-50 column (approx. 3.5 ml) equilibrated with 10mM Tris-HCl (pH 7.5), 1mM EDTA.
2. Collect 5 drops per fraction. Count a 2 microliter aliquot of each fraction by liquid scintillation counting.
3. Pool fractions in the first peak which contain the biotinylated DNA. Discard the second peak which contains nucleotides.
4. Store at 4°C. or -20°C.

Alternatively, DNA may be separated from unincorporated nucleotides using the "spin-column" protocol described below:
1. Pre-swell Sephadex G-50 in 10mM Tris-HCl (pH 7.5), 1mM EDTA and pipette into a 1ml tuberculin syringe that has been plugged with siliconized glass wool. Allow to settle by gravity. Fill the syringe to the top (1.2ml) with resin.
2. Place the filled syringe through a hole cut into the cap of 15 ml plastic conical centrifuge tube. Place a pad of paper towel or kimwipe in the bottom of the conical centrifuge tube and insert a 1.5ml Eppendorf tube. Place the cap with the fitted syringe into the conical centrifuge tube so that the tip of the syringe is fitted into the opening of the Eppendorf tube.
3. Spin the tube-syringe assembly at low speed in a bench top centrifuge for 3-4 minutes. After centrifugation the packed bed volume in the syringe should be between 0.85 and 0.9ml.
4. Remove the syringe and then remove the liquid in the Eppendorf by pipet.
5. Reinsert the syringe into the hole and pipet 50 microliters of the terminal labeling reaction onto the top of the resin bed. The syringe is then recentrifuged for 3-4 minutes at the same speed as initially used to make the packed column.
6. The liquid retained in the Eppendorf contains the DNA probe free of unincorporated nucleotides in a volume equal to or slightly less than that applied (50 microliters). The volume of the sample is adjusted to give a final probe concentration of 20 microgram/ml.

As another alternative, DNA can be isolated by ethanol precipitation by the following procedure:
1. Following end labeling, add an equal volume of 4M ammonium acetate and 20 $\mu$g carrier nucleic acid (20$\mu$l) to the stopped reaction. Mix and then add 2 volumes of ice cold ethanol.
2. Precipitate the DNA fragments by chilling in a dry ice-ethanol bath for 5 minutes. Centrifuge at 12,000 x g for 5 minutes in cold to pellet the DNA. Remove the supernatant carefully with a Pasteur pipe and discard. 3. Add 250 $\mu$l of 0.3M sodium acetate to solubilize the DNA pellet. Add 750ul cold ethanol: chill, centrifuge and remove supernatant as described in Section 2.
4. Repeat step 1.
5. Gently cover the pellet, containing 3' end labeled DNA fragments, with 1ml ethanol. Carefully remove the supernatant and dry the pellet under vacuum for several minutes.
6. Resuspend in desired buffer and determine recovery.

Phenol extraction of biotinylated DNA samples should be avoided because of extraction into the phenol layer or retention at the phenol-water interface.

In the practice of this invention it is preferred to employ as the chemically labeled nucleotide for the terminal labeling of DNA, the biotinylated nucleotide 2-dexoyuridine triphosphate 5-allylamine-biotin referred to hereinabove as biotin-11-dUTP or bio-dUTP. The amount of the chemically labeled nucleotide employed for the terminal labeling of the DNA is usually about 10% of the total deoxynucleotide content so employed. The percentage bio-dUTP, if desired, can be increased up to 100% or decreased to about 1% to produce terminally labeled DNA probes of higher or lower specific activity, respectively. Usually, 10% bio-dUTP content is suitable for most terminal labeling purposes.

As indicated hereinabove, it is to be emphasized that bio-dUTP may be replaced by any of the chemically labeled specially modified deoxynucleotides described or referred to hereinabove.

With respect to the utilization of $^3$H dTTP, this component can be replaced with any other radiolabeled deoxynucleoside triphosphate or, if desired, the radiolabeled nucleoside can be completely omitted. It is pointed out also that the use of more terminal transferase per reaction results in a more rapid synthesis and, conversely, the use of less terminal transferase reduces the rate of reaction or synthesis of the terminally labeled DNA.

It has been found that the concentrations of the components or constituents in the reaction have a marked effect on the resulting terminally labeled probe. For example, adding more or less deoxynucleotide, with time, the terminal tranferase will polymerize up to 90% of the added dCTP or dTTP. Accordingly, as one increases the amount of the added nucleotide longer and longer, "tails" are formed. Also, if the concentration of DNA to be labeled is varied, the number of moles of nucleotide incorporated changes. For example, as the amount of DNA in the reaction increases, a plateau region is reached wherein the total nucleotide incorporation is constant but absolute tail length and numbers of the incorporated chemically labeled nucleotide, e.g. bio-dUTP, per molecule of DNA,decreases. Further, the concentration of metal or metal ion content, e.g. $Co^{++}$ and/or $Mg^{++}$, influences the terminal labeling operation and resulting terminally labeled DNA.

It is mentioned hereinabove that the DNA terminally labeled with the chemically labeled or modified nucleotides can be detected by non-radioactive techniques, such as by means of anti-antibodies directed to the terminally linked special nucleotides or by the use of detectors, such as avidin or streptavidin, or α enzyme-linked avidin or enzyme-linked streptavidin, which would then attach themselves to the biotin component or moiety of the chemically labeled nucleotide. If desired, the terminally linked special nucleotides could be detected by means of radioactive techniques, such as the use of radioactive avidin, to detect or locate terminally linked biotinylated nucleotide in accordance with the practices of this invention.

As mentioned hereinabove, it is preferred in the practices of this invention to employ as the terminally linked nucleotides or as substrate for the terminal transferase, biotinylated nucleotides, either biotin or iminobiotin labeled nucleotides. See the disclosures hereinabove and the nucleotides disclosed in the above-identified Langer et al.publication P.N.A.S. Vol. 78, No. 11, pp. 6633-6637 (1981). Of special interest as the chemically labeled nucleotides are the glycosylated nucleotides. These special nucleotides, which are also capable of being incorporated into double-stranded DNA and employed, as disclosed herein, as substrate for terminal transferase or for terminal linking to DNA, are capable, when terminally linked to DNA, of being readily detected by means of a lectin, such as Concanavalin A. The determination or detection of such glycosylated terminally linked deoxynucleotides can be carried out by employing a radioactive labeled lectin or by means of an antibody or anti-antibody or by means of enzyme-linked lectin in the manner mentioned hereinabove with respect to the detection or determination of biotin-labeled terminally linked nucleotides.

The practices of this invention, as indicated hereinabove, are applicable to the preparation of terminally labeled single-stranded and double-stranded DNA.

Also, as indicated hereinabove, the chemically labeled terminal portion or tails of the DNA molecules or probes in accordance with this invention can be detected by a variety of techniques, such as radioactive detection techniques, enzyme based techniques and immunoassay or antibody based techniques. For example, when the chemically labeled nucleotides making up the tail portion of the DNA molecule contains a biotin moiety, the presence or location of the chemically labeled tail (the biotin moiety) could be detected employing a radioactive avidin or streptavidin or avidin linked to a biotinylated enzyme, such as a complex made up of avidin-biotin-alkaline phosphatase, or streptavidin-biotin-horseradish peroxidase. After attachment of the complex to the biotin moiety of the chemically labeled nucleotide, its presence, for example, would be elicited by a suitable color response or change by the action of the enzyme horseradish peroxidase or alkaline phosphatase on a suitable color generating or color changing substrate. Further, also, the presence of a chemically labeling moiety, such as biotin, in the chemically labeled nucleotide could be elicited or detected by contact with an antibody, such as goat antibody, to biotin and then by contact with a rabbit anti-goat antibody, which would be a linked to an enzyme. A similar approach would be applicable to the detection of a chemically labeled nucleotide wherein the labeled nucleotide making up the tail is glycosylated or includes a glycosidic linkage. Such glycosylated nucleotides could be detected by means of, for example, radioactive lectin, e.g. radioactive Concanavalin A, or by means of other chemicals or antibodies responsive to lectin or enzymes linked to a lectin. Lectin would elicit the presence of a glycosylated nucleotide in accordance with this invention since lectin readily attaches itself to a glycosyl moiety, like avidin readily attaches itself to a biotin moiety. Enzyme-based techniques applicable to the practices of this invention for the detection of the chemically labeled nucleotides are described in copending European Patent Application 84 10 0836.0.

The disclosures of patent application 84 10 0836.0 are herein incorporated and made part of this disclosure. Like the DNA probes described in patent application 84 10 0836.0 the specially terminally labeled DNA or RNA molecules described herein are also capable of being fixed to a transparent substrate, such as glass, and utilized in the manner described in patent application 84 10 0836.0.

Although in the practices of this invention described herein the enzyme DNase has been employed for the preparation of the DNA to be terminally labeled, other enzymes are also usefully employed. For example, restriction endonucleases can be used for the production of 3'-OH termini for subsequent terminal chemical labeling with terminal transferase in accordance with this practice of this invention. Restriction endonuclease Pst 1 which produces protruding 3'-OH termini and restriction endonuclease Bam HI which produces recessed 3'-OH termini have been employed for this purpose. Successful labeling requires an extended incubation with an approximate 2 to 4-fold increase in the amount of terminal transferase in each reaction. Lambda exonuclease, which exposes the recessed 3'-OH termini produced by most restriction endonucleases further enhances the effectiveness of these DNAs as primers for terminal transferase. Flush 3'-OH termini, produced by enzymes such as Hae III, are also suitable. However, restriction enzyme digested DNA must be purified by phenol extraction and ethanol precipitation prior to terminal labeling. For this reason DNase digestion is preferred. Also, randomly sheared DNA, sheared by sonication or other means, can also be terminally labeled. However, there is little or no advantage of this technique over the use of DNase to generate primer termini.

## Claims

1. A reagent kit useful for the preparation of a terminally linked or labeled DNA molecule or probe comprising as reagents therein (1) terminal deoxyribonucleotide transferase TdT (2) DNase and (3) a non-radioactive chemically labeled deoxyribonucleotide capable of acting as a substrate for said TdT.

2. A reagent kit in accordance with claim 1 wherein said non-radioactive chemically labeled deoxyribonucleotide is characterized such that when included in a DNA molecule said chemically labeled deoxyribonucleotide does not prevent said DNA molecule from forming double stranded DNA.

3. A reagent kit in accordance with claim 1 wherein the chemical label moiety of said non-radioactive chemically labeled deoxyribonucleotide is capable of being detected by physical, physcio-chemical, chemical or immunological techniques.

4. A reagent kit in accordance with claim 1 wherein said non-radioactive chemically labeled deoxyribonucleotide is a biotinylated deoxyribonucleotide, especially 2'-deoxyuridine triphosphate 5-allylamine-biotin.

5. A reagent kit in accordance with claim 1 comprising additionally an aqueous $CoCl_2$ and/or $MgCl_2$ solution.

6. A reagent kit in accordance with claim 1 comprising additionally as DNase dilution buffer a solution comprising 10 mM Tris HCl of a pH of about 7.5.

7. A reagent kit in accordance with claim 1 comprising additionally for stabilizing the DNase a solution containing about 1 mg/ml bovine serum albumin.

8. A method of preparing terminally linked or labeled DNA employing the reagent kit of claim 1 which comprises bringing DNA to be terminally linked or labeled, after having been treated with said DNase, into contact with said non-radioactive chemically labeled deoxyribonucleotide in the presence of said TdT to effect terminal attachment of said chemically labeled deoxyribonucleotide to the 3'-OH position presented by said DNA or to any chemically labeled deoxyribonucleotide previously attached to said DNA via said TdT.

9. A reagent kit useful for the preparation of a terminally linked or labeled DNA molecule or probe comprising as reagents therein (1) terminal deoxyribonucleotide transferase TdT (2) an enzyme capable of producing or exposing 3'-OH termini of DNA and (3) a non-radioactive chemically labeled deoxyribonucleotide capable of acting as a substrate for TdT.

**10.** A method of preparing terminally linked or labeled DNA using the reagent kit of claim 9, which method comprises:

treating DNA with an enzyme capable of producing or exposing a 3'-OH terminus of DNA so that a 3'-OH terminus is produced or exposed; and

contacting said treated DNA with a non-radioactive chemically labeled deoxyribonucleotide in the presense of terminal deoxynucleotidyl transferase (TdT) under conditions permitting the attachment of said non-radioactive chemically labeled deoxyribonucleotide to the 3'-OH position or termini presented by said treated DNA or to any non-radioactive chemically labeled deoxyribonucleotide previously attached to said DNA.

**11.** A terminally labeled DNA molecule or probe wherein said DNA molecule or probe is terminally linked or labeled with one or more non-radioactive chemically labeled deoxyribonucleotides which are bound to a 3'-OH terminus of said DNA molecule or probe by terminal deoxynucleotidyl transferase (TdT) wherein said non-radioactive chemically labeled deoxyribonucleotide comprises a glycosylated deoxyribonucleotide.

**12.** A terminally labeled DNA molecule or probe wherein said DNA molecule or probe is terminally linked or labeled with a single stranded polydeoxyribonucleotide bound to a 3'-OH terminus of said DNA molecule or probe by terminal deoxynucleotidyl transferase (TdT), said single stranded polydeoxyribonucleotide containing one or more non-radioactive chemically labeled deoxyribonucleotides.

**13.** A terminally labeled DNA molecule or probe in accordance with claim 12 wherein said non-radioactive chemically labeled deoxyribonucleotide comprises a glycosylated deoxyribonucleotide.

**14.** A terminally labeled DNA molecule or probe in accordance with claim 12 wherein said non-radioactive chemically labeled deoxyribonucleotide comprises a biotinylated deoxyribonucleotide.

**15.** A terminally labeled DNA molecule or probe in accordance with claim 14 wherein said biotinylated deoxyribonucleotide comprises 2'-deoxyuridine triphosphate 5-allylamine-biotin.

**16.** A terminally labeled DNA molecule or probe in accordance with claim 12 wherein from 1% up to 100% of the nucleotides making up said single stranded polydeoxyribonucleotide are said non-radioactive chemically labeled deoxyribonucleotides.

**17.** A terminally labeled DNA molecule or probe in accordance with claim 16 wherein from 2% up to 40% of the nucleotides making up said single stranded polydeoxyribonucleotide are said non-radioactive chemically labeled deoxyribonucleotides.

**Patentansprüche**

**1.** Reagenzkit, verwendbar für die Herstellung eines (einer) terminal verknüpften oder markierten DNA-Moleküls oder - Sonde, umfassend als Reagenzien darin (1) terminale Desoxyribonucleotid-TransferaseTdT, (2) DNase und (3) ein nicht-radioaktives, chemisch markiertes Desoxyribonucleotid, das als Substrat für die TdT wirken kann.

**2.** Reagenzkit gemäß Anspruch 1, wobei das nicht-radioaktive, chemisch markierte Desoxyribonucleotid dadurch gekennzeichnet ist, daß das chemisch markierte Desoxyribonucleotid, wenn es sich in einem DNA-Molekül befindet, das DNA-Molekül nicht daran hindert, eine doppelsträngige DNA zu bilden.

**3.** Reagenzkit gemäß Anspruch 1, wobei die chemische Markierungseinheit des nicht-radioaktiven, chemisch markierten Desoxyribonucleotids durch physikalische, physikalisch-chemische, chemische oder immunologische Methoden nachgewiesen werden kann.

**4.** Reagenzkit gemäß Anspruch 1, wobei das nicht-radioaktive, chemisch markierte Desoxyribonucleotid ein biotinyliertes Desoxyribonucleotid, insbesondere 2'-Desoxyuridintriphosphat-5-allylaminbiotin,ist.

**5.** Reagenzkit gemäß Anspruch 1, zusätzlich eine wäßrige CoCl$_2$- und/oder MgCl$_2$-Lösung umfassend.

8

**6.** Reagenzkit gemäß Anspruch 1, zusätzlich als DNase-Verdünnungspuffer eine Lösung von 10 mM Tris HCl mit einem pH-Wert von etwa 7,5 umfassend.

**7.** Reagenzkit gemäß Anspruch 1, zusätzlich eine Lösung mit einem Gehalt von etwa 1 mg/ml Rinderserumalbumin zur Stabilisierung der DNase umfassend.

**8.** Verfahren zur Herstellung einer terminal verknüpften oder markierten DNA durch Verwendung des Reagenzkits von Anspruch 1, umfassend das Inkontaktbringen der terminal zu verknüpfenden oder zu markierenden DNA, nachdem sie mit der DNase behandelt worden ist, mit dem nicht-radioaktiven chemisch markierten Desoxyribonucleotid in Gegenwart der TdT, so daß eine terminale Verknüpfung des chemisch markierten Desoxyribonucleotids an der 3'-OH-Position der DNA oder an ein beliebiges chemisch markiertes Desoxyribonucleotid, das vorher mittels der TdT an die DNA geknüpft wurde, erfolgt.

**9.** Reagenzkit, verwendbar für die Herstellung eines (einer) terminal verknüpften oder markierten DNA-Moleküls oder -Sonde, umfassend als Reagenzien darin (1) terminale Desoxyribonucleotid-Transferase TdT, (2) ein Enzym, das fähig ist, 3'-OH-Enden der DNA zu erzeugen oder freizulegen und (3) ein nicht-radioaktives, chemisch markiertes Desoxyribonucleotid, das als Substrat für TdT wirken kann.

**10.** Verfahren zur Herstellung von terminal verknüpfter oder markierter DNA unter Verwendung des Reagenzkits nach Anspruch 9, umfassend die folgenden Schritte:
Behandlung von DNA mit einem Enzym, das ein 3'-OH-Ende der DNA erzeugen oder freilegen kann, so daß ein 3'-OH-Ende erzeugt oder freigelegt wird; und
Inkontaktbringen der behandelten DNA mit einem nicht-radioaktiven, chemisch markierten Desoxyribonucleotid in Gegenwart der terminalen Desoxynucleotidyl-Transferase (TdT) unter Bedingungen, die die Anknüpfung des nicht-radioaktiven chemisch markierten Desoxyribonucleotids an die 3'-OH-Position oder -Enden der behandelten DNA oder an ein beliebiges, nicht-radioaktives, chemisch markiertes Desoxyribonucleotid, das vorher an die DNA geknüpft wurde, erlauben.

**11.** Terminal markierte(s) DNA-Molekül oder -Sonde, wobei das (die) DNA-Molekül oder -Sonde terminal verknüpft oder markiert ist mit einem oder mehreren nicht-radioaktiven, chemisch markierten Desoxyribonucleotiden, die an das 3'-OH-Ende des (der) DNA-Moleküls oder -Sonde mit Hilfe der terminalen Desoxynucleotidyltransferase (TdT) gebunden wurden, wobei das nicht-radioaktive, chemisch markierte Desoxyribonucleotid ein glykosyliertes Desoxyribonucleotid umfaßt.

**12.** Terminal markierte(s) DNA-Molekül oder -Sonde, wobei das (die) DNA-Molekül oder -Sonde terminal verknüpft oder markiert ist mit einem einzelsträngigen Polydesoxyribonucleotid, das mit Hilfe des terminalen Desoxynucleotidyltransferase (TdT) an das 3'-OH-Ende des (der) DNA-Moleküls oder -Sonde geknüpft wurde, wobei das einzelsträngige Polydesoxyribonucleotid eines oder mehrere nicht-radioaktive, chemisch markierte Desoxyribonucleotide enthält.

**13.** Terminal markierte(s) DNA-Molekül oder -Sonde gemäß Anspruch 12, wobei das nicht-radioaktive, chemisch markierte Desoxyribonucleotid ein glykosyliertes Desoxyribonucleotid umfaßt.

**14.** Terminal markierte(s) DNA-Molekül oder -Sonde gemäß Anspruch 12, wobei das nicht-radioaktive, chemisch markierte Desoxyribonucleotid ein biotinyliertes Desoxyribonucleotid umfaßt.

**15.** Terminal markierte(s) DNA-Molekül oder -Sonde gemäß Anspruch 14, wobei das biotinylierte Desoxyribonucleotid 2'-Desoxyuridintriphosphat-5-allylaminbiotin umfaßt.

**16.** Terminal markierte(s) DNA-Molekül oder -Sonde gemäß Anspruch 12, wobei 1 bis 100 % der Nucleotide, die das einzelsträngige Polydesoxyribonucleotid ausmachen, die nicht-radioaktiven, chemisch markierten Desoxyribonucleotide bilden.

**17.** Terminal markierte(s) DNA-Molekül oder -Sonde gemäß Anspruch 16, wobei 2 bis 40 % der Nucleotide, die das einzelsträngige Polydesoxyribonucleotid ausmachen, die nicht-radioaktiven, chemisch markierten Desoxyribonucleotide bilden.

**Revendications**

1. Trousse de réactifs utile pour la préparation d'une molécule ou d'une sonde d'ADN à liaison ou marquage terminal, comprenant comme réactifs y inclus (1) de la désoxyribonucléotide-transférase terminale TdT, (2) de la DNase et (3) un désoxyribonucléotide à marquage chimique non-radioactif apte à agir comme substrat pour ladite TdT.

2. Trousse de réactifs selon la revendication 1, dans laquelle ledit désoxyribonucléotide à marquage chimique non-radioactif est tel que, lorsqu'il est inclus dans une molécule d'ADN, ledit désoxyribonucléotide à marquage chimique n'empêche pas ladite molécule d'ADN de former de l'ADN double brin.

3. Trousse de réactifs selon la revendication 1, dans laquelle la partie de marquage chimique dudit désoxyribonucléotide à marquage chimique non-radioactif est apte à être détectée par des techniques physiques, physico-chimiques, chimiques ou immunologiques.

4. Trousse de réactifs selon la revendication 1, dans laquelle ledit désoxyribonucléotide à marquage chimique non-radioactif est un désoxyribonucléotide biotinylé, en particulier de la 2'-désoxyuridine triphosphate 5-allylamine-biotine.

5. Trousse de réactifs selon la revendication 1, comprenant en outre une solution aqueuse de $CoCl_2$ et/ou de $MgCl_2$.

6. Trousse de réactifs selon la revendication 1, comprenant en outre comme tampon de dilution de la DNase une solution comprenant 10 mM de Tris HCl ayant un pH d'environ 7,5.

7. Trousse de réactifs selon la revendication 1, comprenant en outre pour la stabilisation de la DNase une solution contenant environ 1 mg/ml de sérum-albumine bovine.

8. Méthode pour la préparation d'ADN à liaison ou marquage terminal utilisant la trousse de réactifs selon la revendication 1, consistant à amener de l'ADN à comporter une liaison ou un marquage terminal, après avoir été traité avec ladite DNase, en contact avec ledit désoxyribonucléotide à marquage chimique non-radioactif en présence de ladite TdT pour effectuer une fixation terminale dudit désoxyribonucléotide à marquage chimique sur la position 3'-OH présentée par ledit ADN ou sur un quelconque désoxyribonucléotide à marquage chimique précédemment fixé audit ADN via ladite TdT.

9. Trouse de réactifs utile pour la préparation d'une molécule ou d'une sonde d'ADN à liaison ou marquage terminal, comprenant comme réactifs y inclus (1) de la désoxyribonucléotide-transférase terminale TdT, (2) une enzyme apte à produire ou à exposer des extrémités terminales 3'-OH d'ADN et (3) un désoxyribonucléotide à marquage chimique non-radioactif apte à agir comme substrat pour ladite TdT.

10. Méthode pour la préparation d'ADN à liaison ou marquage terminal utilisant la trousse de réactifs selon la revendication 9, comprenant:
le traitement de l'ADN avec une enzyme apte à produire ou à exposer une extrémité terminale 3'-OH d'ADN de telle sorte qu'une extrémité terminale 3'-OH soit produite ou exposée; et
la mise en contact dudit ADN traité avec un désoxyribonucléotide à marquage chimique non-radioactif en présence de désoxyribonucléotidyl-transférase terminale (TdT) dans des conditions permettant la fixation dudit désoxyribonucléotide à marquage chimique non-radioactif sur la position 3'-OH ou les extrémités terminales présentées par ledit ADN traité ou sur un quelconque désoxyribonucléotide à marquage chimique non-radioactif précédemment fixé audit ADN.

11. Molécule ou sonde d'ADN à marquage terminal, dans laquelle ladite molécule ou sonde d'ADN comporte une fixation ou un marquage terminal avec un ou plusieurs désoxyribonucléotides à marquage chimique non-radioactif, qui sont liés à une extrémité terminale 3'-OH de ladite molécule ou sonde d'ADN par de la désoxyribonucléotidyl-transférase (TdT) et dans laquelle ledit désoxyribonucléotide à marquage chimique non-radioactif comprend un désoxyribonucléotide glycosylé.

**12.** Molécule ou sonde d'ADN à marquage terminal, dans laquelle ladite molécule ou sonde d'ADN comporte une fixation ou un marquage terminal avec un polydésoxyribonucléotide simple brin lié à une extrémité terminale 3'-OH de ladite molécule ou sonde d'ADN par de la désoxyribonuclétidyl-transférase (TdT) terminale, ledit polydésoxyribonucléotide simple brin contenant un ou plusieurs désoxyribonucléotides à marquage chimique non-radioactif.

**13.** Molécule ou sonde d'ADN à marquage terminal selon la revendication 12, dans laquelle ledit désoxyribonucléotide à marquage chimique non-radioactif comprend un désoxyribonucléotide glycosylé.

**14.** Molécule ou sonde d'ADN à marquage terminal selon la revendication 12, dans laquelle ledit désoxyribonucléotide à marquage chimique non-radioactif comprend un désoxyribonucléotide biotinylé.

**15.** Molécule ou sonde d'ADN à marquage terminal selon la revendication 14, dans laquelle ledit désoxyribonucléotide biotinylé comprend de la 2'-désoxyuridine triphosphate 5-allylamine-biotine.

**16.** Molécule ou sonde d'ADN à marquage terminal selon la revendication 12, dans laquelle de 1% jusqu'à 100% des nucléotides formant ledit polydésoxyribonucléotide simple brin sont lesdits désoxyribonucléotides à marquage chimique non-radioactif.

**17.** Molécule ou sonde d'ADN à marquage terminal selon la revendication 16, dans laquelle de 2% jusqu'à 40% des nucléotides formant ledit polydésoxyribonucléotide simple brin sont lesdits désoxyribonucléotides à marquage chimique non-radioactif.